# EUROPEAN PATENT SPECIFICATION

(11) **EP 1 835 902 B1**
(45) Date of publication and mention of the grant of the patent: **11.03.2009**
(21) Application number: 05815907.0
(22) Date of filing: 22.12.2005
(51) Int. Cl.: A61K 31/357, C07D 319/08

(54) **SPIRO[BORNYL-2,4'-(1,3-DIOXANES)] AND THEIR USES AS FRAGRANCE INGREDIENTS**
SPIRO[BORNYL-2,4'-(1,3-DIOXANE)] UND IHRE VERWENDUNGEN ALS DUFTINHALTSSTOFFE
SPIRO[BORNYL-2,4'-(1,3-DIOXANNES)] ET LEURS UTILISATIONS COMME INGREDIENTS DE FRAGRANCE

(30) Priority: 04.01.2005 GB 0500007
(43) Date of publication of application: 26.09.2007
(73) Proprietor: Givaudan SA, 1214 Vernier-Genève (CH)
(72) Inventor: BAJGROWICZ, Jerzy, A., CH-8053 Zürich (CH)
(74) Representative: Givaudan Patents
(86) International application number: PCT/CH2005/000769
(87) International publication number: WO 2006/072189

(56) References cited:
- EP-A1- 0 761 664

## Description

The present invention relates to substituted spiro[bornyl-2,4'-(1,3-dioxanes)]. This invention relates furthermore to a method for their production and to fragrance compositions comprising them.

In the fragrance industry there is a constant demand for new compounds that enhance or improve on odour, or impart new odour notes. Particularly preferred are those compounds possessing a low odour threshold and that thus may be used in fragranced products at lower concentration than compounds of similar odour profile having a higher odour threshold without essentially influencing the character of a fragrance composition comprising it.

EP 0 761 664 refers to spiro[bornyl-2,4'-(1,3-dioxane)] derivatives substituted at 2' position of the dioxane ring optionally substituted with a methyl group at 6' position of the dioxane ring. The compounds described therein possess woody and ambery notes, often accompanied with camphoraceous, patchouli aspects.

It has now been found that the odour threshold can positively be influenced if the compounds described in EP 0 761 664 are substituted at C5' of the dioxane ring. This class of compounds has not been described in the literature and thus is novel in its own right. Accordingly, the present invention refers in one of its aspects to a compound of formula (1) 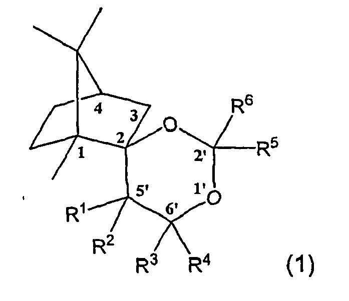 wherein R¹ to R⁶ independently represent hydrogen; C₁₋₄ alkyl, e.g. methyl, ethyl, *n-*propyl, *i*-propyl, and *i*-butyl; or C₂₋₄ alkenyl, e.g. vinyl, allyl, propenyl and *i*-propenyl; or C₃-₄ cycloalkyl, e.g. cyclopropyl; or at least one of the residues R¹ and R², R³ and R⁴, and/or R⁵ and R⁶ form together with the carbon atom to which they are attached a C₃₋₅ cycloalkyl ring; with the proviso that R¹ is C₁₋₄ alkyl, C₂₋₄ alkenyl or ; or C₃-₄ cycloalkyl if R² is hydrogen; and the number of carbon atoms of R¹+R²+R³+R⁴+R⁵+R⁶ is between 1 and 6, preferably between 2 and 5, most preferably between 2 and 4.

The compounds of formula (1) comprise several chiral centres and as such may exist as a mixture of stereoisomers, or they may be resolved as isomerically pure forms. Resolving stereoisomers adds to the complexity of manufacture and purification of these compounds and so it is preferred to use the compounds as mixtures of their stereoisomers simply for economic reasons. However, if it is desired to prepare individual stereoisomers, this may be achieved according to methods known in the art, e.g. preparative HPLC and GC, crystallization or stereoselective synthesis.

Preferred are compounds of formula (1) wherein R¹ is methyl or ethyl, or compounds
wherein R¹ is methyl or ethyl and R³ and/or R⁴ is methyl. Most preferred are compounds
wherein R¹ is methyl and R², R³ and R⁴ is hydrogen, or compounds wherein R¹ and R³ is methyl and R² and R⁴ is hydrogen, or compounds wherein R¹, R⁴ and R³ is methyl and R² is hydrogen.

Particularly preferred are compounds selected from the list comprising
(1R,2S,2'S,4R,5'S)-1,2',5',7,7-pentamethylspiro[bicyclo[2.2.1]heptane-2,4'-[1,3]dioxane], (1R,2S,2'R,4R,5'S)-1,2',5',7,7-pentamethylspiro[bicyclo[2.2.1]heptane-2,4'-[1,3]dioxane], (1 R,2S,2'S,4R,5'R)-1,2',5',7,7-pentamethylspiro[bicyclo[2.2.1]heptane-2,4'-[1,3]dioxane], (1R,2S,2'S,4R,5'S)-2'-ethyl-1,5',7,7-tetramethylspiro[bicyclo[2.2.1]heptane-2,4'-[1,3]dioxane], (1R,2S,2'R,4R,5'S)-2'-ethyl-1,5',7,7-tetramethylspiro[bicyclo[2.2.1]heptane-2,4'-[1,3]dioxane], (1R,2S,2'S,4R,5'S)-2'-isopropyl-1,5',7,7-tetramethylspiro[bicyclo[2.2.1]heptane-2,4'-[1,3]dioxane], (1R,2S,2'R,4R,5'S)-2'-isopropyl-1,5',7,7-tetramethylspiro[bicyclo[2.2.1]heptane-2,4'-[1,3]dioxane], (1 R,2S,2'S,4R,5'R)-2'-isopropyl-1,5',7,7-tetramethylspiro[bicyclo[2.2.1]heptane-2,4'-[1,3]dioxane], (1R,2S,4R,5'S)-1,2',2',5',7,7-hexamethylspiro[bicyclo[2.2.1]heptane-2,4'-[1,3]dioxane], (1R,2S,4R,5'R)-1,2',2',5',7,7-hexamethylspiro[bicyclo[2.2.1]heptane-2,4'-[1,3]dioxane], (1 RS,2SR,2'RS/SR,4RS,5'RS/SR,6'RS/SR)-1,2',5',6',7,7-hexamethylspiro[bicyclo[2.2.1]heptane-2,4'-[1,3]dioxane], (1 RS,2SR,4RS,5'RS/SR,6'RS/SR)-1,2',2',5',6',7,7-heptamethylspiro[bicyclo[2.2.1]heptane-2,4'-[1,3]dioxane], (1RS,2SR,2'RS/SR,4RS,5'RS/SR,6'RS/SR)-6'-Ethyl-1,2',5',7,7-pentamethylspiro[bicyclo[2.2.1]heptane-2,4'-[1,3]dioxane], (1RS,2SR,4RS,5'RS/SR,6'RS/SR)-6'-ethyl-1,2',2',5',7,7-hexamethylspiro[bicyclo[2.2.1]heptane-2,4'-[1,3]dioxane] and (1RS,2SR,2'RS/SR,4RS,5'RS/SR)-5'-Ethyl-1,2',7,7-tetramethylspiro[bicyclo[2.2.1]heptane-2,4'-[1,3]dioxane].

Compounds of formula (1) wherein R¹ and R⁵ is not hydrogen and R² and R⁶ is hydrogen are preferred from an olfactive point of view, if the mixture of stereoisomers is enriched in (1 R,2S,2'S,5'S)-stereoisomer, e.g. diastereomer mixture of (1RS,2SR,2'RS/SR,4RS,5'RS/SR)-1,2',5',7,7-pentamethytspiro[bicyclo[2.2.1]heptane-2,4'-[1,3]dioxane] enriched in (1R,2S,2'S,4R,5'S)-1,2',5',7,7-pentamethylspiro[bicyclo[2.2.1]heptane-2,4'-[1,3]dioxane], is preferred. Thus, in a further aspect, the present invention refers to the use of a compound of formula (1) enriched in a compound of formula (1') 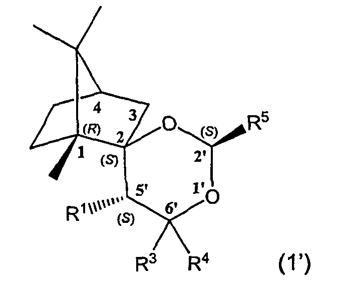 wherein R¹ and R³ to R⁵ have the same meaning as given above.

The term "enriched" is used herein to describe a compound containing more than 25 weight % of the (1R,2S,2'S,5'S)-stereoisomer. Compounds are preferred containing 50 weight % or more of the (1 R,2S,2'S,5'S)-stereoisomer. Particularly preferred are compounds having a diastereomeric purity of 75 weight %, or greater.

The compounds according to the present invention may be used alone or in combination with a base material. As used herein, the "base material" indues all known odourant molecules selected from the extensive range of natural products and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odourants in fragrance compositions, for example, carrier materials, and other auxiliary agents commonly used in the art.

The following list comprises examples of known odourant molecules, which may be combined with the compounds of the present invention:
- ethereal oils and extracts, e.g. tree moss absolute, basil oil, fruit oils such as bergamot oil and mandarine oil, myrtle oil, palmarose oil, patchouli oil, petitgrain oil, jasmine oil, rose oil, sandalwood oil, wormwood oil, lavender oil or ylang-ylang oil;
- alcohols, e.g. cinnamic alcohol, cis-3-hexenol, citronellol, Ebanol^{™}, eugenol, farnesol, geraniol, Super Muguet^{™}, linalool, menthol, nerol, phenylethyl alcohol, rhodinol, Sandalore^{™}, terpineol or Limberol^{™}.
- aldehydes and ketones, e.g. anisaldehyde, α-amylcinnamaldehyde, Georgywood^{™}, hydroxycitronellal, Iso E Super^{®}, Isoraldeine^{®}, Hedione^{®}, Lilial^{®}, maltol, Methyl cedryl ketone, methylionone, verbenone or vanillin;
- ethers and acetals, e.g. Ambrox^{™}, geranyl methyl ether, rose oxide or Spirambrene^{™}.
- esters and lactones, e.g. benzyl acetate, Cedryl acetate, γ-decalactone, Helvetolide^{®}, γ-undecalactone or Vetivenyl acetate.
- macrocycles, e.g. Ambrettolide, Ethylene brassylate or Exaltolide^{®}.
- heterocycles, e.g. isobutylchinoline.

The compounds according to formula (1) may be used in a broad range of fragrance applications, e.g. in any field of fine and functional perfumery, such as perfumes, household products, laundry products, body care products and cosmetics. The compounds can be employed in widely varying amounts, depending upon the specific application and on the nature and quantity of other odourant ingredients. The proportion is typically from 0.001 to 20 weight percent of the application. In one embodiment, compounds of the present invention may be employed in a fabric softener in an amount of from 0.001 to 0.05 weight percent. In another embodiment, compounds of the present invention may be used in fine perfumery in amounts of from 0.1 to 20 weight percent, more preferably between 0.1 and 5 weight percent. However, these values are given only by way of example, since the experienced perfumer may also achieve effects or may create novel accords with lower or higher concentrations.

The compounds of the present invention may be employed into the fragrance application simply by directly mixing the fragrance composition with the fragrance application, or they may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the application.

Thus, the invention additionally provides a method of manufacturing a fragrance application, comprising the incorporation of a compound of the present invention, as a fragrance ingredient, either by directly admixing the compound to the application or by admixing a fragrance composition comprising a compound of formula (1), which may then be mixed to a fragrance application, using conventional techniques and methods.

As used herein, "fragrance application" means any product, such as fine perfumery, e.g. perfume and eau de toilette; household products, e.g. detergents for dishwasher, surface cleaner; laundry products, e.g. softener, bleach, detergent; body care products, e.g. shampoo, shower gel; and cosmetics, e.g. deodorant, vanishing creme, comprising an odourant. This list of products is given by way of illustration and is not to be regarded as being in any way limiting.

The compounds of the present invention may be prepared by reaction of the corresponding diol of the general formula (2) 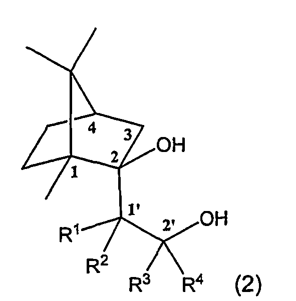 with a carbonyl compound of the formula R⁵R⁶C=O, or an acetal or ketal of the formula R⁵R⁶C(OR"')₂, wherein R¹ to R⁶ have the same meaning as defined for the compound of formula (1), and R'" is methyl, ethyl or propyl, under conditions known in the art.

The diol of the general formula (2) may be obtained either via substituted vinylisoborneol **4** (route I) or allylisoborneol **5** (route II and III) as depicted in Scheme 1. Both isoborneols may be prepared by addidion of a corresponding organometallic reagent, preferably Grignard reagent, to camphor. In route II and III, the allylisoborneol **5** undergoes either epoxidation or ozonolysis, both followed by reduction or addition of an organometallic reagent. In route I, the vinylisoborneol **4** is hydroborated and oxidized, to give the diol of formula (2).

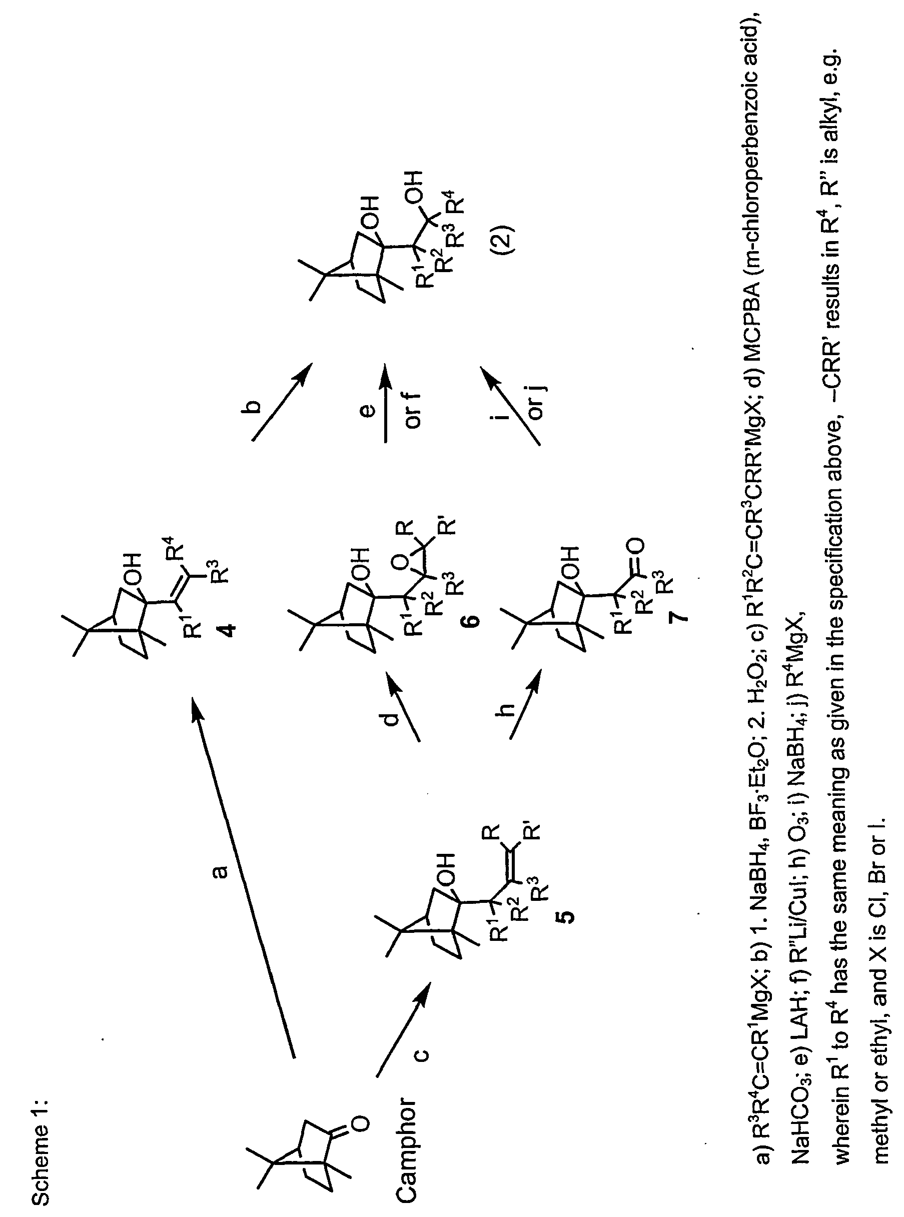

Whereas the procedure according to route II and III are generally known, route I has not been described in literature.

Surprisingly, it has been found that the compounds of the present invention wherein the mixture of stereoisomers is enriched in those of configuration (1R,2S,2'S,5'S) is obtained if the diol of formula (2) has been prepared following the procedure according to route I, as described above.

Further particulars as to reaction conditions are provided in the examples.

The invention is now further described with reference to the following non-limiting

### examples.

The reported NMR data were measured under the following general conditions: ¹H at 400 and ¹³C at 100 MHz; in CDCl₃, if not otherwise stated; chemical shifts (δ) in ppm downfield from TMS; coupling constants J In Hz; NOESY, and GRASP COSY-DQF, HMBC and HMQC data were used in signal attributions. The reported MS and GC/MS data refer to the intensities (in brackets) in % rel. to the base peak. GC: DB^{™}-1701. Flash chromatography: *Merck* silica gel 60 (230 - 400 mesh), if not otherwise stated.
The new products were obtained as colourless oils, except where otherwise stated. The yields were not optimised.

### Example 1: (1R,2S,2'R/S,4R,5'S)-1,2',5',7,7-Pentamethylspiro[bicyclo[2.2.1]heptane-2,4'-[1,3]dioxane] (1a)

### a) (1R,25,4R)2-Isopropenyl-1,7,7-trimethylbicyclo[2.2.1]heptan-2-ol (4a)

A small amount of 2-bromopropene was added to magnesium turnings (6.0 g, 0.25 mol) covered with minimum of THF. After starting the reaction, a solution of 2-bromopropene (total amount 27 ml, 0.30 mol) in THF (200 ml) was added dropwise in a rate to maintain gentle reflux of the reaction mixture. After additional 30 min. of reflux, a suspension prepared by 2 h stirring of (1 R)-(+)-camphor (20 g, 0.13 mol) and dried cerium (III) chloride (10 g, 41 mmol) in THF (100 ml) was added at room temperature. Stirring at rt continued for 4 h, then the reaction mixture was poured into aqueous solution of ammonium chloride (300 ml) and extracted with MTBE (2 x 200 ml). After washing with brine, drying (MgSO₄), evaporation of solvents in vacuo and sublimation of the unreacted camphor, crude (1R,2S,4R)-2-isopropenyl-1,7,7-trimethylbicyclo[2.2.1]heptan-2-ol (**4a**, 24 g, quantitative yield, 95% GC pure) was obtained. It was engaged in the next step without further purification.
¹H-NMR: δ0.85 (s, 3H), 0.99 (s, 3H), 1.03 (ddd, *J*=12.3, 8.8, 5.3, 1H), 1.14 (s, 3H), 1.27 (ddd, *J*=13.1, 8.8, 4.1, 1H), 1.34 (ddd, *J*=13.1, 11.2, 5.3, 1H), 1.57 (s, 1H), 1.61-1.70 (m, 1H), 1.74 (t, *J*=4.3, 1H), 1.88 (dd, *J*=1.4, 0.5, 3H), 1.92 (ddd, *J*=13.6, 4.3, 3.3, 1H), 2.08 (d, *J*=13.6, 1H), 4.90 (qi, *J*=1.4, 1H), 5.06 (sb, 1H); ¹³C-NMR: δ11.6 (q), 21.4 (q), 21.5 (q), 21.8 (q), 26.7 (t), 30.8 (t), 42.9 (t), 45.1 (d), 50.0 (s), 52.3 (s), 83.9 (s), 112.1 (t), 149.8 (s); MS: 195 (0.5), 194 (4, M⁺), 179 (3), 161 (3), 133 (5), 123 (4), 110 (18), 109 (30), 108 (10), 95 (100), 85 (11), 84 (15), 69 (15), 67 (8), 55 (9), 43 (10), 41 (20); [α]_{D}²² -54.9 (c 1.0, EtOH).

### b) (1 R,2S,2'S,4R)- 2-(2-Hydroxy-1-methylethyl)-1,7,7-trimethylbicyclo[2.2.1]heptan-2-ol (2a) and (1R,2S,2'R,4R)- 2-(2-hydroxy-1-methylethyl)-1,7,7-trimethylbicyclo[2.2.1]heptan-2-ol (2b)

A solution of (1R,2S,4R)-2-isopropenyl-1,7,7-trimethylbicyclo[2.2.1]heptan-2-ol (**4a**, 23 g, 0.12 mol) in diglyme (46 ml) was added to a suspension of sodium borohydride (6.7 g, 0.18 mol) in the same solvent (46 ml). Boron trifluoride diethyl etherate (23 ml, 0.18 mol) was added to the reaction mixture cooled with an ice bath and stirring continued for 1 h. 4N sodium hydroxide (53 ml, 0.21 mol) and 30% aqueous hydrogen peroxide (21 ml, 0.21 mmol) solutions were added successively while maintaing the temperature below 20°C. After further 45 min. stirring at room temperature, the reaction mixture was diluted with water (100 ml) and extracted with MTBE (2 x 300 ml). The combined organic layers were washed with water (300 ml), aqueous sodium bicarbonate solution (100 ml) and again with water (2 x 200 ml). After drying (MgSO₄) and evaporation of solvents in vacuo, the residue was purified by flash chromatography (n-hexane/MTBE 2:1) to give (1 R,2S,2'S,4R)- 2-(2-hydroxy-1-methylethyl)-1,7,7-trimethylbicyclo[2.2.1]heptan-2-ol (**2a**, 17.1 g, 68%), its epimer (1 R,2S,2'R,4R)- 2-(2-hydroxy-1-methylethyl)-1,7,7-trimethylbicyclo[2.2.1]heptan-2-ol (**2b**, 3.3 g, 13%), and a 9:10 mixture of **2a** and **2b** (3.8 g, 15%).

### c) (1R,2S,2'R/S,4R,5'S)-1,2',5',7,7-Pentamethylspiro[bicyclo[2.2.1]heptane-2,4'-[1,3]dioxane] (1a)

A solution of diol **2a** (3.0 g, 14 mmol), acetaldehyde (2.2 g, 50 mmol) and p-toluenesulfonic acid monohydrate (0.20 g, 1.1 mmol) in anhydrous THF (45 ml) was stirred overnight under nitrogen at room temperature, then poured into ice-cold 10 % NaHCO₃ solution (100 ml) and extracted with MTBE (2 x 100 ml). The combined organic phases were washed with brine (150 ml), dried (MgSO₄) and concentrated in vacuo. The residue (3.3 g) was purified by flash chromatography (n-hexane/MTBE 20:1) to give a 1:1.1 mixture of (1 R,2S,2'S,4R,5'S)- and (1 R,2S,2'R,4R,5'S)-1,2',5',7,7-pentamethylspiro[bicyclo[2.2.1]heptane-2,4'-[1,3]dioxane] (**1a,** 2.6 g, 77%).
[α]_{D}²² -49.0 (*c* 1.1, EtOH).

For the analytical data of single epimers see Example 2, compounds 1 band 1 c.

Odour description (mixture of epimers): woody, ambery, fruity, rich, sweet.

### Example 2: (1R,2S,2'S,4R,5'S)-1,2,5',7,7-Pentamethylspiro[bicyclo[2.2.1]heptane-2,4'-[1,3]dioxane] (1b) and (1 R,2S,2'R,4R,5'S)-1,2',5',7,7-pentamethylspiro[bicyclo[2.2.1]heptane-2,4'-[1,3]dioxane] (1c)

Samples of single stereoisomer **1b** (first eluted) and **1c** were obtained by flash chromatography separation (n-hexane/MTBE 40:1) of the epimeric mixture **1a** obtained in Example 1.
**1b:** ¹H NMR: δ0.86 (s, 3H), 0.87 (d, *J*=6.6, 3H), 0.88 (s, 3H), 0.99 (s, 3H), 0.99-1.09 (m, 1H), 1.20 (d, *J*=5.1, 3H), 1.36 (ddd, *J*=13.9, 11.9, 5.6, 1H), 1.48 (d, *J*=13.4, 1H), 1.58 (ddd, *J*=13.9, 9.0, 3.3, 1H), 1.62-1.72 (m, 1H), 1.76 (t, *J*=4.5, 1H), 1.95 (ddd, *J*=13.4, 4.5, 3.0, 1H), 2.10 (ddq, *J*=11.6, 6.6, 5.1, 1H), 3.20 (t, *J*=11.6, 1H), 3.67 (dd, *J*=11.6, 5.1, 1H), 4.72 (q, *J*=5.1, 1H); ¹³C NMR: δ 10.9 (q), 15.4 (q), 20.8 (q), 21.3 (q), 21.8 (q), 26.3 (t), 29.8 (t), 31.4 (d), 33.6 (t), 45.1 (d), 49.8 (s), 54.9 (s), 71.2 (t), 86.6 (s), 90.7 (d); MS: 239 (0.3), 238 (2, M⁺), 194 (7), 179 (11), 164 (6), 152 (29), 137 (11), 123 (14), 121 (22), 110 (20), 109 (53), 108 (100), 95 (59), 93 (23), 81 (31), 69 (20), 55 (27), 43 (38), 41 (52); [α]_{D}²² -47.6 (*c* 1.0, EtOH).

Odour description: ambery, woody, dry, sweet, animalic.
**1c:** ¹H NMR: δ0.82 (s, 3H), 0.93-1.01 (m, 1H), 1.09 (d, *J*=6.7, 3H), 1.09 (s, 3H), 1.11 (s, 3H), 1.18 (d, *J*=5.1, 3H), 1.24-1.34 (m, 1H), 1.49 (ddd, *J*=14.0, 9.5, 3.4, 1H), 1.61-1.69 (m, 2H), 1.64 (d, *J*=13.9, 1H), 1.82 (ddd, *J*=13.9, 4.0, 3.5, 1H), 2.29 (qid, *J*=6.7, 3.3 1H), 3.55 (dd, *J*=10.6, 6.8 1H), 3.90 (dd, *J*=10.6, 3.0, 1H), 5.32 (q, *J*=5.1, 1H); ¹³C NMR: δ 15.4 (q), 17.1 (q), 21.1 (q), 21.2 (q), 21.6 (q), 26.9 (t), 30.8 (t), 31.2 (d), 42.0 (t), 44.2 (d), 50.0 (s), 54.8 (s), 68.6 (t), 86.1 (s), 92.2 (d); MS: 239 (0.5), 238 (3, M⁺), 194 (8), 179 (14), 164 (6), 152 (12), 137 (13), 123 (18), 121 (21), 110 (19), 109 (61), 108 (100), 95 (67), 93 (25), 81 (25), 69 (21), 55 (30), 43 (45), 41 (57); [α]_{D}²² -49.5 (c 1.1, EtOH).

Odour description: dry, woody, cedarwood, ambery, warm, powdery, weaker than **1b.**

### Example 3: (1R,2S,2'S,4R,5'R)-1,2',5',7,7-Pentamethylspiro[bicyclo[2.2.1]heptane-2,4'-[1,3]dioxane] (1d)

Prepared according to Example 1c starting from diol **2b**, described in Example 1 b. The evaluated sample contained about 4% of tentatively (1 R,2S,2'R,4R,5'R) epimer.
¹H NMR: δ0.82 (s, 3H), 0.93 (s, 3H), 0.94 (s, 3H), 0.94-1.03 (m, 1H), 1.24 (d, *J*=5.1, 3H), 1.245 (d, *J*=6.1, 3H), 1.25 (d, *J*=13.0, 1H), 1.28-1.35 (m, 1H), 1.35-1.42 (m, 1H), 1.49 (ddd, *J*=13.2, 9.5, 3.4, 1H), 1.67-1.76 (m, 2H), 2.33 (ddd, *J*=13.0, 3.9, 3.7, 1H) 3.66 (dd, *J*=11.2, 1.7, 1H) 4.02 (dd, *J*=1.2, 1.7, 1H), 4.88 (q, *J*=5.1, 1H); ¹³C NMR: δ11.8 (q), 15.9 (q), 20.8 (q), 20.9 (q), 21.4 (q), 27.4 (t), 28.3 (t), 37.9 (d), 42.1 (t), 44.7 (d), 50.3 (s), 52.3 (s), 71.6 (t), 83.9 (s), 91.3 (d); MS: 239 (1), 238 (7, M⁺), 194 (18), 179 (12), 152 (37), 137 (12), 123 (16), 121 (15), 110 (27), 109 (62), 108 (100), 95 (80), 93 (26), 81 (52), 69 (36), 55 (34), 45 (27), 43 (40), 41 (62); [α]_{D}²² -65.3 (c 0.6, EtOH).

Odour description: ambery, woody, balsamic, green.

### Example 4: (1RS,2SR,2'RS/SR,4RS,5'RS/SR)-1,2',5',7,7-Pentamethylspiro[bicyclo[2.2.1]heptane-2,4'-[1,3]dioxane] (1e and 1f)

a) Starting from rac-camphor and according to the experimental procedure of Example 1a-c, except for the separation of the intermediate diols **2c,** a 1:14:10 (sorted by GC elution order) racemic mixture **1e** of (1R*,2S*,2'S*,4R*,5'R*)-, (1R*,2S*,2'S*,4R*,5'S*)-and (1R*,2S*,2'R*,4R*,5'S*)-diastereomers of 1,2,5',7,7-pentamethylspiro[bicyclo[2.2.1]heptane-2,4'-[1,3]dioxane] was obtained in 82% overall yield.
   Odour description: ambery, woody, earthy/mossy, fruity.
b) A 6:2:1 racemic mixture **1f** of diastereomers of (1R*,2S*,2'S*,4R*,5'R*)-, (1R*,2S*,2'S*,4R*,5'S*)- and (1R*,2S*,2'R*,4R*,5'S*)-1,2',5',7,7-pentamethylspiro[bicyclo[2.2.1]heptane-2,4'-[1,3]dioxane] was obtained in 38% overall yield following the experimental procedure of Example 1 c, starting from the diol mixture **2d** obtained from rac-camphor via 2-(1-methylallyl)-1,7,7-trimethylbicyclo[2.2.1]heptan-2-ol (**5a**), applying the methodology of Kostova et al (Helv. Chim. Acta 1999, 82, 1385), i.e. route III. The epimeric intermediate enols and diols were not separated. (1R*,2S*,2'S*,4R*,5'S*)- and (1R*,2S*,2'S*,4R*,5'R*)- enatiomeric pairs are the strongest constituents of the mixture. Their relative odour threshold, compared to the whole stereoisomer mixture, were 1:50:4 (rac-**1b** : rac-**1d** : **1f**).

Odour description (**1f**): woody, fruity, ambery.

### Example 5 (compounds 1g - 1n):

The following dioxanes were obtained starting from diols **2a, 2b, 2c** or **2d** described in Examples 1b and 4, following the experimental procedures of Example 1c and 2:

### a) (11R,2S,2'S,4R,5'S)-2'-Ethyl-1,5',7,7-tetramethylspiro[bicyclo[2.2.1]heptane-2,4'-[1,3]dioxane] (1g)

¹H NMR (C₆D₆): δ0.55 (d, *J*=6.6, 3H), 0.85 (s, 3H), 0.88-0.93 (m, 1H), 0.97 (t, *J*= 7.6, 3H), 1.02 (s, 3H), 1.14 (s, 3H), 1.23-1.30 (m, 1H), 1.26 (d, *J*=13.4, 1H), 1.45 (ddd, *J*=13.9, 9.0, 3.3, 1H), 1.53-1.75 (m, 4H), 1.80 (ddd, *J*=13.4, 4.5, 3.0, 1H), 2.00 (ddq, *J*=11.4, 6.6, 5.1, 1H), 3.01 (t, *J*=11.4, 1H), 3.59 (dd, *J*=11.4, 5.1, 1H), 4.44 (t, J=4.8, 1H); ¹³C NMR (C₆D₆): δ8.3 (q), 10.9 (q), 15.2 (q), 20.7 (q), 22.2 (q), 26.2 (t), 28.6 (t), 29.8 (t), 31.5 (d), 33.5 (t), 45.2 (d), 49.7 (s), 54.9 (s), 70.8 (t), 86.4 (s), 94.6 (d); MS: 253 (0.3), 252 (2, M⁺), 194 (5), 179 (10), 164 (44), 152 (12), 149 (27), 135 (24), 122 (20), 121 (100), 109 (44), 108 (87), 107 (61), 95 (42), 93 (96), 79 (39), 58 (38), 55 (34), 41 (73), 29 (68); [α]_{D}²² -53.7 (c 1.0, EtOH).

Odour description: woody, ambery, green, sweet, floral.

### b) (1R,2S,2'R,4R,5'S)-2'-Ethyl-1,5',7,7-tetramethylspiro[bicyclo[2.2.1]heptane-2,4'-[1,3]dioxane] (1h)

¹H NMR (C₆D₆): δ0.76-0.86 (m, 1H), 0.78 (s, 3H), 0.95 (d, *J*=6.6, 3H), 0.99 (t, *J*= 7.6, 3H), 1.02 (s, 3H), 1.10-1.20 (m, 1H), 1.26 (s, 3H), 1.28 (ddd, *J*=13.9, 9.4, 3.5, 1H), 1.49-1.58 (m, 2H), 1.52 (d, *J*=13.6, 1H), 1.68 (m, 2H), 1.90-1.98 (m, 2H), 3.48 (dd, *J*=10.6, 6.3, 1H), 3.75 (dd, *J*=10.6, 3.0, 1H), 5.12 (t, *J*=4.8, 1H); ¹³C NMR (C₆D₆): δ8.3 (q), 15.5 (q), 17.1 (q), 21.1 (q), 21.5 (q), 27.0 (t), 29.4 (t), 31.0 (t), 31.5 (d), 42.5 (t), 44.4 (d), 50.1 (s), 54.7 (s), 68.3 (t), 85.8 (s), 96.4 (d); MS: 253 (0.5), 252 (3, M⁺), 194 (7), 179 (14), 164 (45), 149 (27), 135 (24), 122 (22), 121 (100), 109 (53), 108 (95), 107 (63), 95 (53), 93 (100), 91 (46), 79 (41), 77 (31), 67 (28), 58 (41), 55 (39), 41 (81), 29 (73); [α]_{D}²² +22.9 (c 0.9, EtOH).

Odour description: woody, green, weaker than compound **1g.**

### c) (1R,2S,2'S,4R,5'S)-2'-Isopropyl-1,5',7,7-tetramethylspiro[bicyclo[2.2.1]heptane-2,4'-[1,3]dioxane] (1i)

¹H NMR (C₆D₆): δ0.55 (d, *J*=6.6, 3H), 0.85 (s, 3H), 0.85-0.98 (m, 1H), 1.00 (s, 3H), 1.045 (d, *J*=7.1, 3H), 1.05 (d, *J*=7.1, 3H), 1.14 (s, 3H), 1.22-1.30 (m, 1H), 1.26 (d, *J*=13.4, 1H), 1.44 (ddd, *J*=13.9, 9.1, 3.3, 1H), 1.53-1.61 (m, 1H), 1.65 (t, *J*=4.5, 1H), 1.80 (ddd, *J*=13.4, 4.5, 3.3, 1H), 1.86 (qid, *J*=6.8, 4.0, 1H), 1.97 (ddq, *J*=11.4, 6.6, 5.1, 1H), 3.00 (t, *J*=11.4, 1H), 3.60 (dd, *J*=11.4, 5.1, 1H), 4.28 (d, *J*=4.0, 1H); ¹³C NMR (C₆D₆): δ10.9 (q), 15.3 (q), 17.1 (q), 17.3 (q), 20.8 (q), 22.6 (q), 26.2 (t), 30.0 (t), 31.6 (d), 33.3 (d), 33.6 (t), 45.3 (d), 49.7 (s), 55.2 (s), 70.8 (t), 86.4 (s), 97.2 (d); MS: 267 (0.4), 266 (2, M⁺), 194 (7), 179 (15), 177 (26), 164 (24), 152 (16), 149 (16), 135 (17), 121 (67), 109 (45), 108 (97), 107 (43), 95 (53), 93 (65), 81 (34), 79 (28), 69 (30), 55 (38), 43 (84), 41 (100); [α]_{D}²² -52.5 (c 0.7, EtOH).

Odour description: woody, ambery, sweet, dry, cedarwood, creamy.

### d) (1R,2S,2'R,4R,5'S)-2'-Isopropyl-1,5',7,7-tetramethylspiro[bicyclo[2.2.1]heptane-2,4'-[1,3]dioxane] (1j)

¹H NMR (C₆D₆): δ0.78 (s, 3H), 0.80-87 (m, 1H), 0.95 (d, *J*=6.6, 3H), 1.04 (s, 3H), 1.05 (d, *J*=6.8, 3H), 1.08 (d, *J*=6.8, 3H), 1.10-1.18 (m, 1H), 1.22-1.32 (m, 1H), 1.25 (s, 3H), 1.49-1.57 (m, 2H), 1.52 (d, *J*=13.9, 1H), 1.86 (qid, *J*=6.8,4.5, 1H), 1.87-1.96 (m, 2H), 3.46 (dd, *J*=10.6, 6.3, 1H), 3.76 (dd, *J*=10.6, 2.8, 1H), 4.96 (d, *J*=4.5, 1H); ¹³C NMR (C₆D₆): δ15.7 (q), 16.7 (q), 17.1 (q), 17.2 (q), 21.2 (q), 21.7 (q), 27.0 (t), 31.1 (t), 31.5 (d), 34.0 (d), 42.6 (t), 44.4 (d), 50.1 (s), 54.8 (s), 68.4 (t), 85.8 (s), 98.8 (d); MS: 267 (0.7), 266 (4, M⁺), 194 (9), 179 (19), 177 (17), 164 (24), 152 (8), 149 (14), 135 (15), 121 (59), 109 (49), 108 (100), 107 (39), 95 (59), 93 (62), 81 (29), 79 (27), 69 (27), 55 (37), 43 (79), 41 (92); [α]_{D}²² +18.4 (c 1.1, EtOH).

Odour description: ambery, woody, green, floral, weaker than compound **1i.**

### e) (1R,2S,2'S,4R,5'R)-2'-Isopropyl-1,5',7,7-tetramethylspiro[bicyclo[2.2.1]heptane-2,4'-[1,3]dioxane] (1k)

¹H NMR (C₆D₆): δ0.81 (s, 3H), 0.81-0.93 (m, 2H), 0.95 (d, *J*=13.1, 1H), 1.03 (d, *J*=7.1, 3H), 1.04 (s, 3H), 1.05 (s, 3H), 1.06 (d, *J*=7.1, 3H), 1.22 (d, *J*=7.1, 3H), 1.24-1.39 (m, 2H), 1.57-1.67 (m,2H), 1.89 (qid, *J*=7.1, 4.1, 1H), 2.15 (dt, *J*=13.1, 3.8, 1H), 3.49 (dd, *J*=11.1, 1.5, 1H), 3.72 (dd, *J*=11.1, 1.5, 1H), 4.39 (d, *J*=4.1, 1H); ¹³C NMR: δ11.7 (q), 15.8 (q), 16.5 (q), 17.5 (q), 20.9 (q), 21.2 (q), 27.4 (t), 28.4 (t), 32.9 (d), 38.2 (d), 42.0 (t), 44.6 (d), 50.2 (s), 52.7 (s), 71.4 (t), 83.6 (s), 97.7 (d); MS: 267 (1), 266 (5, M⁺), 194 (17), 179 (13), 177 (29), 153 (12), 152 (30), 137 (10), 123 (14), 121 (28), 109 (51), 108 (100), 95 (63), 93 (33), 81 (44), 69 (32), 69 (30), 55 (38), 43 (54), 41 (68); [α]_{D}²² -62.5 (*c* 0.7, EtOH).

Odour description: fruity, animalic, woody, touch ambrette seeds.

### f) (1RS,2SR,2'RS/SR,4RS,S'RS/SR)-2'-Ethyl-1,5',7,7-tetramethylspiro[bicyclo[2.2.1]-heptane-2,4'-[1,3]dioxane] (1I)

1:1.6:1.7 (GC elution order) racemic mixture of diastereomers.

Odour description: woody, ambery, fruity, carrot, sweet.

### g) (1RS,2SR,2'RS/SR,4RS,5'SR)-2'-Isopropyl-1,5',7,7-tetramethylspiro[bicyclo[2.2.1]-heptane-2,4'-[1,3]dioxane] (1m)

1:1.2 (1R*,2S*,2'S*, 4R*,5'S*)/(1R*,2S*,2'R*,4R*,5'S*) racemic mixture of epimers.

Odour description: ambery, woody, fruity, rich.

### h) (1 RS,2SR,2'RS/SR,4RS,5'RS/SR)-2'-Isopropyl-1,5',7,7-tetramethylspiro [bicyclo[2.2.1]heptane-2,4'-[1,3]dioxane] (1n)

2.3:27:1.2:1 (GC elution order) racemic mixture of diastereomers.

Odour description: fruity, woody, green.

### Example 6: (1R,2S,4R,5'S)-1,2',2',5',7,7-Hexamethylspiro[bicyclo[2.2.1]heptane-2,4'-[1,3]dioxane] (1o)

A solution of diol **2a** from Example 1 b (3.0 g, 14 mmol), 2,2-dimethoxypropane (2.35 g, 23 mmol), PTSA monohydrate (0.20 g, 1.1 mmol) and lithium bromide (0.20 g, 2.3 mmol) in THF (45 ml) was stirred overnight at room temperature. The reaction mixture was poured into ice-cold aqueous sodium bicarbonate solution (100 ml), and extracted with MTBE (2 x 100 ml). After washing with brine (100 ml), drying (MgSO₄) and evaporation of solvents in vacuo, the residue was purified by flash chromatography (neutral aluminum oxide; n-hexane/MTBE 15:1) to give (1R,2S,4R,5'S)-1,2',2',5',7,7-hexamethylspiro[bicyclo[2.2.1]heptane-2,4'-[1,3]dioxane] (**1o,** 0.99 g, 28%).
¹H NMR: δ 0.84 (s, 3H), 0.845 (s, 3H), 0.98 (d, *J*=6.8, 3H), 0.99-1.06 (m, 1H), 1.07 (s, 3H), 1.26 (s, 3H), 1.32 (ddd, *J*=13.9, 11.6, 5.1, 1H), 1.43 (s, 3H), 1.46 (ddd, *J*=13.9, 9.1, 3.5, 1H), 1.57-1.67 (m, 1H), 1.66 (d, *J*=13.6, 1H), 1.69 (t, *J*=4.5, 1H), 2.02-2.12 (m, 1H), 2.09 (ddd, *J*=13.6, 4.5, 3.0, 1R), 3.40 (dd, *J*=11.8, 7.6, 1H), 3.65 (dd, *J*=11.8, 5.3, 1H); ¹³C NMR: δ10.9 (q), 17.5 (q), 21.0 (q), 21.7 (q), 24.9 (q), 26.3 (t), 29.2 (q), 29.4 (t), 30.9 (d), 37.9 (t), 45.8 (d), 49.3 (s), 55.6 (s), 64.9 (t), 84.7 (s), 97.4 (s); MS: 253 (0.1), 252 (1, M⁺), 194 (15), 179 (21), 164 (21), 149 (13), 137 (10), 135 (13), 123 (13), 121 (55), 109 (45), 108 (100), 107 (36), 95 (78), 93 (58), 81 (40), 69 (29), 59 (40), 55 (34), 43 (100), 41 (67); [α]_{D}²² -43.5 (c 1.0, EtOH).

Odour description: woody, ambery, camphoraceous, earthy/mossy.

### Example 7: (1R,2S,4R,5'R)-1,2',2',5',7,7-Hexamethylspiro[bicyclo[2.2.1]heptane-2,4'-[1,3]dioxane] (1p)

Prepared according to the experimental procedure of Example 6 starting from diol 2b of Example 1 b.
¹NMR: δ 0.81 (s, 3H), 0.89 (s, 3H), 0.94-1.00 (m, 1H), 1.01 (s, 3H), 1.23 (d, *J*=6.6, 3H), 1.31-1.38 (m, 2H), 1.33 (s, 3H), 1.38 (d, *J*=12.6, 1H), 1.47 (s, 3H), 1.54 (ddd, *J*=12.9, 9.3, 3.5, 1H), 1.62-1.73 (m, 1H), 1.65 (t, *J*=4.5, 1H), 2.54 (ddd, *J*=12.6, 4.5, 3.3, 1H), 3.49 (dd, *J*=11.6,2.3, 1H), 4.28 (dd, *J*=11.6, 1.6, 1H); ¹³C NMR: δ12.1 (q), 16.2 (q), 20.9 (q), 24.1 (q), 27.2 (t), 28.4 (t), 31.3 (q), 31.3 (q), 37.6 (d), 45.0 (d), 45.4 (t), 50.4 (s), 52.9 (s), 65.2 (t), 83.4 (s), 98.0 (s); MS: 253 (0.1), 252 (1, M⁺), 194 (28), 179 (18), 177 (12); 164 (15), 152 (9), 149 (10), 137 (10), 135 (11), 125 (17), 121 (45), 109 (48), 108 (100), 107 (30), 95 (82), 93 (48), 81 (46), 69 (35), 59 (44), 55 (34), 43 (83), 41 (64); [α]_{D}²² -68.1 (c 1.0, EtOH).

Odour description: woody, agrestic, ambery, earthy/mossy, dry, cedarwood aspect.

### Example 8: (1RS,2SR,4RS,5'RS/SR)-1,2',2',5',7,7-Hexamethylspiro[bicyclo[2.2.1] heptane-2,4'-[1,3]dioxane] (1q)

Prepared according to the experimental procedure of Example 6 starting from diols **2d** of Example 4b as a 1:1.8 (1R*,2S*,4R*,5'S*)/(1R*,2S*,4R*,5'R*) racemic mixture of epimers.

Odour description: earthy/mossy, woody, vetiver aspect.

### Example 9: (1RS,2SR,2'RS/SR,4RS,5'RS/SR,6'RS/SR)-1,2',5',6',7,7-Hexamethylspiro[bicyclo[2.2.1]heptane-2,4'-[1,3]dioxane] (1r)

### a) (1 RS,2SR,1'RS/SR,2'RS/SR,4RS)- 2-(2-Hydroxy-1-methylpropyl)-1,7,7-trimethylbicyclo[2.2.1]heptan-2-ol (2e)

A diastereoisomer mixture of (1 RS,2SR,4RS)-2-(1-oxiranylethyl)-1,7,7-trimethylbicyclo[2.2.1]heptan-2-ols was prepared according to the procedure described by Dimitrov, V.; Philipova, I. and Simova, S. (Tetrahedron: Asymmetry 1996, 7, 1493), i.e. route II, by MCPBA oxidation of (1RS,2SR,1'RS/SR,4RS)-2-(1-methylallyl)-1,7,7-trimethylbicyclo[2.2.1]heptan-2-ol (**5a**) cited in Example 4b. A solution of this mixture (20 g, 89 mmol) in diethyl ether (60 ml) was added dropwise to lithium aluminum hydride (3.4 g, 89 mmol) suspended in the same solvent (200 ml), at t <10°C. The reaction mixture was stirred at room temperature for 2.5 h, then more LAH (3.4 g, 89 mmol) was added and stirring continued for 0.5 h. An aqueous 2M solution of sodium hydroxide (17 ml, 34 mmol) was added under ice bath cooling, the solid filtered off and the filtrate washed with brine (200 ml), dried (MgSO₄) and concentrated in vacuo to give crude racemic mixture of diastereomers of (1 RS,2SR,1'RS/SR,2'RS/SR,4RS)-2-(2-hydroxy-1-methylpropyl)-1,7,7-trimethylbicydo[2.2.1]heptan-2-ol (**2e**, 17.5 g, 87%, waxy white solid) used in the next step without further purification.

### b) (1RS,2SR,2'RS/SR,4RS,5'RS/SR,6'RS/SR)-1,2',5',6',7,7-hexamethylspiro[bicyclo[2.2.1]heptane-2,4'-[1,3]dioxane] (1r)

Prepared as a 3.2:13.6:1:1.3 (GC elution order) racemic mixture of diastereoisomers starting from the above described diol, according to the experimental procedure of Example 1c.
¹H NMR (main racemic diastereoisomer): δ0.82 (s, 3H), 0.94 (s, 3H), 0.94-1.20 (m, 1H), 0.95 (s, 3H), 1.01 (d, *J*=6.6, 3H), 1.13-1.19 (m, 1H), 1.14 (d, *J*=6.6, 3H), 1.22 (d, *J*=13.7, 1H), 1.25 (d, *J*=5.1, 3H), 1.33-1.43 (m, 1H), 1.47-1.55 (m, 1H), 1.67-1.76 (m, 1H), 1.69 (t, *J=*4.5, 1H), 2.30 (dt, *J*=12.9, 3.8, 1H), 3.98 (qd, *J*=6.6,2.0, 1H), 4.90 (q, *J*= 5.1, 1H); ¹³C NMR (main racemic diastereoisomer): δ9.4 (q), 12.1 (q), 19.0 (q), 20.9 (q), 21.0 (q), 21.4 (q), 27.5 (t), 28.2 (t), 41.8 (d), 41.9 (t), 44.6 (d), 50.0 (s), 52.5 (s), 72.5 (d), 84.9 (s), 91.2 (d); MS (main racemic diastereoisomer): 253 (0.5), 252 (2, M⁺), 208 (23), 193 (10), 153 (30), 152 (21), 137 (7), 121 (11), 110 (28), 109 (59), 108 (98), 95 (100), 93 (29), 91 (25), 83 (31), 81 (81), 79 (28), 69 (39), 67 (31), 56 (45), 55 (48), 43 (35), 41 (61).
Odour description: Woody, fruity, ambery, sweet, cedarwood.

### Example 10: (1RS,2SR,4RS,5'RS/SR,6'RS/SR)-1,2',2',5',6',7,7-Heptamethylspiro[bicyclo[2.2.1]heptane-2,4'-[1,3]dioxane] (1s)

Prepared as a 1:5.5 (GC elution order) racemic mixture of epimers starting from diol **2e** described in Example 9a, following the experimental procedure of Example 6.
¹H NMR (main racemic epimer): δ0.81 (s, 3H), 0.82 (s, 3H), 0.94-1.02 (m, 1H), 0.99 (d, *J*=6.6, 3H), 1.02 (s, 3H), 1.12-1.18 (m, 1H), 1.14 (d, *J*=6.6, 3H), 1.31-1.40 (m, 1H), 1.33 (s, 3H), 1.36 (d, *J*=12.6, 1H), 1.46 (s, 3H), 1.55 (ddd, *J*=13.4, 9.3, 3.5, 1H), 1.63-173 (m, 1H), 1.64 (t, *J*=4.4, 1H), 2.51 (ddd, *J*=12.6, 4.4, 3.3, 1H), 4.27 (qd, *J*=6.4, 1.4, 1H); ¹³C NMR: (main racemic diastereoisomer): δ9.5 (q), 12.4 (q), 19.3 (q), 20.9 (q), 21.0 (q), 24.6 (q), 27.4 (t), 28.3 (t), 31.4 (q), 41.8 (d), 44.9 (d), 45.2 (t), 50.5 (s), 52.9 (s), 65.6 (d), 84.9 (s), 98.5 (s); MS (main racemic diastereoisomer): 266 (0.1, M⁺), 251 (1), 208 (24), 191 (11), 153 (10), 152 (19), 135 (10), 121 (16), 110 (27), 109 (57), 108 (93), 95 (100), 93 (34), 83 (32), 81 (76), 67 (27), 59 (43), 55 (48), 43 (64), 41 (57).
Odour description: Woody, earthy/mossy, cedarwood.

### Example 11: (1 RS,2SR,2'RS/SR,4RS,5'RS/SR,6'RS/SR)-6'-Ethyl-1,2',5',7,7-pentamethylspiro[bicyclo[2.2.1]heptane-2,4'-[1,3]dioxane] (1t)

### a) (1RS,2SR,1'RS/SR,2'RS/SR,4RS)- 2-(2-Hydroxy-1-methylbutyl)-1,7,7-trimethylbicyclo[2.2.1]heptan-2-ol (2f)

1.6 M solution of methyl iodide in diethyl ether (223 ml, 0.36 mol) was added to a suspension of copper (I) iodide (34 g, 0.18 mol) in diethyl ether (100 ml) at t <-20°C and stirring continued at this temperature for 35 min. The clear yellow reaction mixture was cooled to -40°C and the crude 2-(1-oxiranylethyl)-1,7,7-trimethylbicyclo[2.2.1]heptan-2-ol from Example 9a (5.0 g, 22 mmol) dissolved in diethyl ether (25 ml) was added. After stirring for 1 h at -40°C and 2 h at -20°C, the reaction mixture was poured into an ice-cold solution of ammonium chloride (300 ml), acidified with 2M HCl and filtered through Celite^{®}. The filtrate was extracted with MTBE (2 x 200 ml), washed with brine (2 x 200 ml) dried (MgSO₄) and concentrated in vacuo to give crude (1 RS,2SR, 1'RS/SR,2'RS/SR,4RS)- 2-(2-hydroxy-1-methylbutyl)-1,7,7-trimethylbicyclo[2.2.1]heptan-2-ol (**2f**, 4.2 g, 79%, waxy white solid), used in the next ketalisation steps without further purification.

### b) (1RS,2SR,2'RS/SR,4RS,5'RS/SR,6'RS/SR)-6'-Ethyl-1,2',5',7,7-pentamethylspiro[bicyclo[2.2.1]heptane-2,4'-[1,3]dioxane] (1t)

Prepared as a 1.6:3.8:1 (GC elution order) racemic mixture of diastereoisomers starting from the above described diol (**2f**), according to the experimental procedure of Example 1 c.
¹³C NMR (main racemic diastereoisomer): δ 9.6 (q), 10.0 (q), 12.2 (q), 20.9 (q), 21.0 (q), 21.4 (q), 25.7 (t), 27.6 (t), 28.2 (t), 40.1 (d), 42.0 (t), 44.6 (d), 50.5 (s), 52.5 (s), 78.7 (d), 84.9 (s), 91.3 (d); MS (main racemic diastereoisomer): 267 (0.4), 266 (2, M⁺), 237 (2), 222 (18), 193 (28), 153 (51), 152 (18), 135 (7), 121 (12), 110 (29), 109 (64), 108 (100), 95 (98), 93 (32), 81 (78), 70 (38), 69 (55), 55 (58), 43 (32), 41 (61).
Odour description: woody, fruity, green, pine, eucalyptus, cedarwood.

### Example 12: (1RS,2SR,4RS,5'RS/SR,6'RS/SR)-6'-Ethyl-1,2',2',5',7,7-hexamethylspiro[bicyclo[2.2.1]heptane-2,4'-[1,3]dioxane] (1u)

Prepared as a 1:2 racemic mixture of diastereoisomers starting from diol **2f** described in Example 11a, following the experimental procedure of Example 6.
¹H NMR (main racemic epimer, C₆D₆): δ0.85 (s, 3H), 0.86-0.95 (m, 1H), 0.90 (t, *J*=7.4, 3H), 1.06-1.09 (m, 4H), 1.08 (s, 3H), 1.18 (s, 3H), 1.18-1.27 (m, 1H), 1.19 (d, *J*=12.7, 1H), 1.28-1.35 (m, 1H), 1.40 (s, 3H), 1.45 (s, 3H), 1.47-1.54 (m, 1H), 1.59 (t, *J*=4.6, 1H), 1.59-1.68 (m, 2H), 2.45 (ddd, *J*=12.7, 4.6, 3.0, 1H), 3.85 (dd, *J*=8.2, 5.1, 1H); ¹³C NMR: (main racemic diastereoisomer, C₆D₆): δ10.3 (q), 10.4 (q), 12.9 (q), 21.2 (q), 21.5 (q), 24.6 (q), 26.2 (t), 27.7 (t), 28.6 (t), 31.7 (q), 40.6 (d), 45.2 (d), 45.6 (t), 50.8 (s), 53.2 (s), 71.8 (d), 85.1 (s), 98.8 (s); MS: 280 (0.1, M⁺), 265 (1), 222 (14), 205 (10), 193 (15), 153 (28), 152 (20), 135 (8), 121 (14), 110 (29), 109 (66), 108 (99), 95 (100), 93 (31), 83 (26), 81 (80), 80 (25), 69 (55), 67 (29), 59 (53), 55 (52), 43 (56), 41 (57).
Odour description: floral, sweet, woody, cedarwood aspect.

### Example 13: (1RS,2SR,2'RS/SR,4RS,5'RS/SR)-5'-Ethyl-1,2',7,7-tetramethylspiro[bicyclo[2.2.1]heptane-2,4'-[1,3]dioxane] (1v)

### a) (1RS,2SR,4RS)-2-(1-Methylenepropyl)-,7,7-trimethylbicyclo[2.2.1]heptan-2-ol (4b)

Prepared following the experimental procedure of Example 1 a starting from racemic camphor and 2-bromobut-1-ene.
¹H-NMR: δ0.85 (s, 3H), 0.97 (s, 3H), 1.04 (ddd, *J*=12.2, 9.0, 5.1, 1H), 1.08 (t, *J*=7.3, 3H), 1.15 (s, 3H), 1.22 (ddd, *J*=13.1, 9.0, 4.1, 1H), 1.31 (ddd, *J*=13.1, 11.4, 5.1, 1H), 1.57 (s, 1H), 1.61-1.69 (m, 1H), 1.74 (t, *J*=4.4, 1H), 1.93 (ddd, *J*=13.6, 4.3, 3.3, 1H), 2.08 (d, *J*=13.6, 1H), 2.05-2.16 (m, 1H), 2.20-2.30 (m, 1H), 4.94 (sb, 1H), 5.18 (sb, 1H). ¹³C-NMR: δ11.4 (q), 13.7 (q), 21.4 (q), 21.5 (q), 25.9 (t), 26.8 (t), 30.8 (t), 43.1 (t), 45.0 (d), 50.0 (s), 52.6 (s), 84.4 (s), 109.6 (t), 155.8 (s); MS: 209 (0.2), 208 (1, M⁺), 193 (2), 190 (11), 175 (12), 147 (20), 133 (14), 119 (14), 110 (14), 109 (25), 108 (10), 99 (16), 95 (100), 91 (16), 83 (27), 69 (10), 55 (32), 43 (16), 41 (26).

### b) (1RS,2SR,1'RS/SR,4RS/SR)-2-(1-Hydroxymethylpropyl)-1,7,7-trimethyl-bicyclo[2.2.1]heptan-2-ol (2g)

Prepared as a racemic mixture of epimers following the experimental procedure of Example 1b starting from **4b**. The crude (1RS,2SR,1'RS/SR,4RS/SR)-2-(1-hydroxymethylpropyl)-1,7,7-trimethyl-bicyclo[2.2.1]heptan-2-ol (**2g,** waxy solid) was used in the next step without further purification.

### c) (1RS,2SR,2'RS/SR,4RS,5'RS/SR)-5'-Ethyl-1,2',7,7-tetramethylspiro[bicyclo[2.2.1]heptane-2,4'-[1,3]dioxane] (1v)

Prepared as a 1:1.3:1.2 (GC elution order) racemic mixture of diastereoisomers starting from (1RS,2SR,1'RS/SR,4RS/SR)-2-(1-hydroxymethylpropyl)-1,7,7-trimethyl-bicyclo[2.2.1]heptan-2-ol (**2g**), according to the experimental procedure of Example 1c.
¹H NMR (main racemic diastereoisomer (1R*,2S*,2'S*,4R*,5'S*), C₆D₆): δ0.48 (sp, *J*=7.4, 1H), 0.72 (t, *J*=7.4, 3H), 0.86 (s, 3H), 0.92-1.01 (m, 1H), 1.03 (s, 3H), 1.13 (s, 3H), 1.18 (d, *J*=13.4, 1H), 1.27-1.39 (m, 3H), 1.28 (d, *J*=5.1, 3H), 1.58-1.71 (m, 2H), 1.66 (t, *J*=4.6, 1H), 1.88 (ddd, *J*=13.4, 4.6, 3.0, 1H), 3.08 (dd, *J*=11.4, 0.8, 1H), 3.87 (dd, *J*=11.4, 5.3, 1H), 4.66 (q, *J*=5.1, 1H); ¹³C NMR (main racemic diastereoisomer, C₆D₆): δ11.3 (q), 13.5 (q), 20.9 (q), 21.6 (q), 22.3 (q), 22.6 (t), 27.2 (t), 29.7 (t), 34.7 (t), 39.8 (d), 45.4 (d), 50.1 (s), 55.1 (s), 69.4 (t), 85.9 (s), 91.2 (d); MS (main racemic diastereoisomer): 253 (0.4), 252 (2, M⁺), 208 (5), 179 (12), 164 (6), 153 (15), 152 (34), 135 (25), 125 (23), 121 (19), 110 (21), 109 (59), 108 (100), 107 (23), 95 (66), 93 (33), 81 (41), 69 (29), 56 (39), 55 (53), 43 (58), 41 (76), 29 (35).
Odour description: woody, ambery, fruity, slightly carrot-like.

### Example 14: Determination of GC-odour threshold concentration

According to standard procedures known to the person skilled in the art, threshold concentrations for volatile perfumery compounds are determined on a gas chromatograph equipped with a sniff port by a panel of trained evaluators. The lowest concentration smelled by each panellist is recorded as the individual threshold concentration expressed in ng (absolute amount of compound delivered at the sniff port).

Under identical conditions the odour threshold concentration for racemic mixture of compounds A, B and C respectively was measured and compared by a group of 4 panelists. The results are given below.

| | |
|---|---|
| 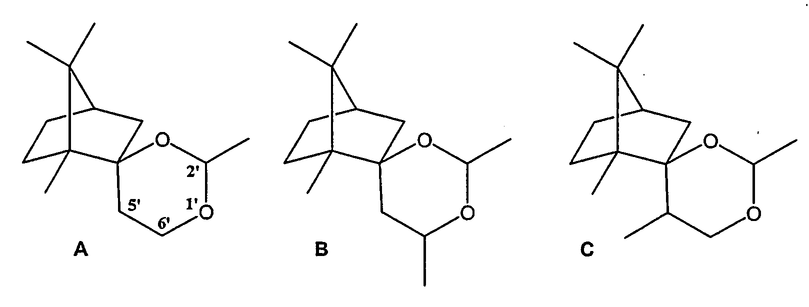 | |

| Compound | odour threshold concentration [ng] geometric mean |
|---|---|
| **A** (Example la of EP 0 761 664) | 22 |
| **B** (Example Is of EP 0 761 664) | 68 |
| **C** (compound according to the present invention wherein R¹ and R⁵ is methyl and R², R³, R⁴ and R⁶ is hydrogen) | 3 |

It can be seen from the results that the compounds of the present invention which is substituted at C5' position has an odour threshold value which is more than 22 times lower compared to the compound substituted at C6' position and more than 7 times
lower compared to the compound which is substituted at C2' position only. With other-words, whereas the substitution at C6' position (prior art) negatively influence the odour threshold, the substitution at C5' position influence it positively compared to the compound which is substituted neither at C5' nor at C6' position (prior art). Based on this, a significant advance is achieved because much smaller amounts of the claimed compounds is required to impart the same odour.

### Example 15: Feminine fine fragrance composition

| | |
|---|---|
| | Parts per weight |
| Ambrofix | 12 |
| Cashmeran | 20 |
| Cepionate | 150 |
| Cyclamen aldehyde | 150 |
| Damascone alpha | 4 |
| Dihydromyrcenol | 100 |
| Florhydral | 13 |
| Galaxolide 50 PHT | 170 |
| Gardenol | 20 |
| Geraniol | 80 |
| Grisalva | 1 |
| Heliotropine | 50 |
| Hydroxycitronellal | 65 |
| Peonile | 65 |
| Phenoxanol | 50 |
| Compound **1b** (from Example 2) | 50 |
| | 1000 |

In this feminine accord, the presence of the compound **1b** enhances the woody amber notes and makes the whole composition richer, more dynamic and more elegant.

## Claims

1. A compound of formula (1) 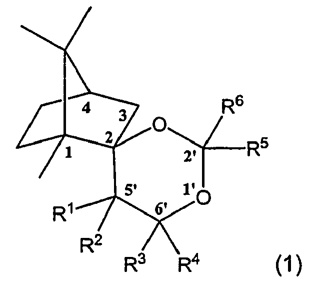 wherein R¹ to R⁶ independently represent hydrogen; C₁₋₄ alkyl; or C₂₋₄ alkenyl; or C₃₋₄ cycloalkyl; or at least one of the residues R¹ and R², R³ and R⁴, and/or R⁵ and R⁶ form together with the carbon atom to which they are attached a C₃₋₅ cycloalkyl ring; with the proviso that R¹ is C₁₋₄ alkyl, C₂₋₄ alkenyl, or C₃₋₄ cycloalkyl if R² is hydrogen; and the number of carbon atoms of R¹+R²+R³+R⁴+R⁵+R⁶ is between 1 and 6.

2. A compound according to claim 1 wherein R² and R⁶ is hydrogen and the compound of formula (1) is enriched in a compound of formula (1') 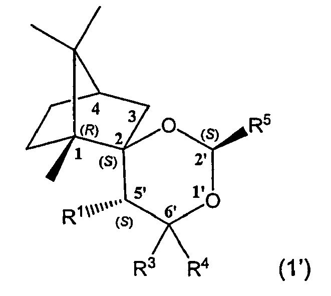 wherein R¹ and R⁵ is C₁₋₄ alkyl; or C₂₋₄ alkenyl; or C₃₋₄ cycloalkyl; and R³ and R⁴ have the same meaning as given in claim 1.

3. A compound according to claim 1 selected from the group consisting of
(1R,2S,2'S,4R,5'S)-1,2',5',7,7-pentamethylspiro[bicyclo[2.2.1]heptane-2,4'-[1,3]dioxane],
(1R,2S,2'R,4R,5'S)-1,2',5',7,7-pentamethylspiro[bicyclo[2.2.1]heptane-2,4'-[1,3]dioxane],
(1R,2S,2'S,4R,5'R)1,2',5',7,7-pentamethylspiro[bicyclo[2.2.1]heptane-2,4'-[1,3]dioxane],
(1R,2S,2'S,4R,5'S)-2'-ethyl-1,5',7,7-tetramethylspiro[bicyclo[2.2.1]heptane-2,4'-[1,3]dioxane],
(1R,2S,2'R,4R,5'S)-2'-ethyl-1,5',7,7-tetramethylspiro[bicyclo[2.2.1]heptane-2,4'-[1,3]dioxane],
(1R,2S,2'S,4R,5'S)-2'-isopropyl-1,5',7,7-tetramethylspiro[bicyclo[2.2.1]heptane-2,4'-[1,3]dioxane],
(1R,2S,2'R,4R,5'S)-2'-isopropyl-1,5',7,7-tetramethylspiro[bicyclo[2.2.1]heptane-2,4'-[1,3]dioxane],
(1R,2S,2'S,4R,5'R)-2'-isopropyl-1,5',7,7-tetramethylspiro[bicyclo[2.2.1]heptane-2,4'-[1,3]dioxane],
(1R,2S,4R,5'S)-1,2',2',5',7,7-hexamethylspiro[bicyclo[2.2.1]heptane-2,4'-[1,3]dioxane],
(1R,2S,4R,5'R)-1,2',2',5',7,7-hexamethylspiro[bicyclo[2.2.1]heptane-2,4'-[1,3]dioxane],
(1RS,2SR,2'RS/SR,4RS,S'RS/SR,6'RS/SR)-1,2',5',6',7,7-hexamethylspiro[bicyclo[2.2.1]heptane-2,4'-[1,3]dioxane],
(1RS,2SR,4RS,5'RS/SR,6'RS/SR)-1,2',2',5',6',7,7-heptamethylspiro[bicyclo[2.2.1]heptane-2,4'-[1,3]dioxane],
(1RS,2SR,2'RS/SR,4RS,5'RS/SR,6'RS/SR)-6'-Ethyl-1,2',5',7,7-pentamethylspiro[bicyclo[2.2.1]heptane-2,4'-[1,3]dioxane],
(1RS,2SR,4RS,5'RS/SR,6'RS/SR)-6'-ethyl-1,2',2',5',7,7-hexamethylspiro[bicyclo[2.2.1]heptane-2,4'-[1,3]dioxane] and
(1RS,2SR,2'RS/SR,4RS,5'RS/SR)-5'-Ethyl-1,2',7,7-tetramethylspiro[bicyclo[2.2.1]heptane-2,4'-[1,3]dioxane].

4. A fragrance composition comprising a compound as defined in one of the preceding claims.

5. The use as fragrance ingredient of a compound as defined in one of the claims 1 to 3.

6. A method of manufacturing a fragrance application, comprising the incorporation of an effective amount of a compound of formula (1) as defined in one of the claims 1 to 3.

## Patentansprüche

1. Verbindung der Formel (1) 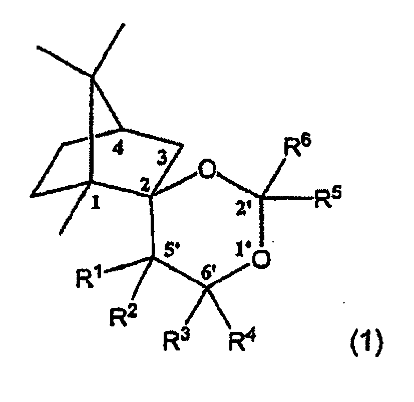 worin R¹ bis R⁶ unabhängig voneinander für Wasserstoff; C₁₋₄-Alkyl oder C₂₋₄-Alkenyl oder C₃₋₄-Cycloalkyl stehen oder mindestens einer der Reste R¹ und R², R³ und R⁴ und/oder R⁵ und R⁶ gemeinsam mit dem Kohlenstoffatom, an das sie gebunden sind, einen C₃₋₅-Cycloalkylring bilden; mit der Maßgabe, daß R¹ für C₁₋₄-Alkyl, C₂₋₄-Alkenyl oder C₃₋₄-Cycloalkyl steht, wenn R² für Wasserstoff steht; und die Zahl der Kohlenstoffatome von R¹+R²+R³+R⁴+R⁵+R⁶ zwischen 1 und 6 liegt.

2. Verbindung nach Anspruch 1, worin R² und R⁶ für Wasserstoff stehen und die Verbindung der Formel (1) an einer Verbindung der Formel (1') 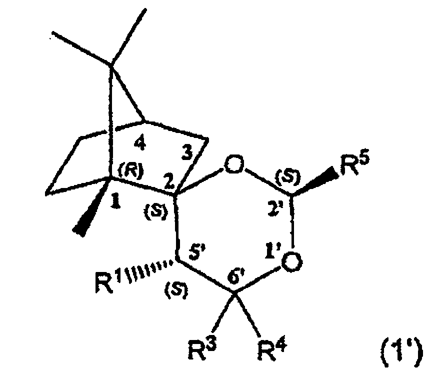 worin R¹ und R⁵ für C₁₋₄-Alkyl oder C₂₋₄-Alkenyl oder C₃₋₄-Cycloalkyl stehen und
R³ und R⁴ die gleiche Bedeutung wie in Anspruch 1 besitzen;
angereichert ist.

3. Verbindung nach Anspruch 1, ausgewählt aus der Gruppe bestehend aus
(1R,2S,2'S,4R,5'S)-1,2',5',7,7-Pentamethylspiro-[bicyclo[2.2.1]heptan-2,4'-[1,3]dioxan];
(1R,2S,2'R,4R,5'S)-1,2',5',7,7-Pentamethylspiro-[bicyclo[2.2.1]heptan-2,4'-[1,3]dioxan];
(1R,2S,2'S,4R,5'R)-1,2',5',7,7-Pentamethylspiro-[bicyclo[2.2.1]heptan-2,4'-[1,3]dioxan];
(1R,2S,2'S,4R,5'S)-2'-Ethyl-1,5',7,7-tetramethyl-spiro[bicyclo[2.2.1]heptan-2,4'-[1,3]dioxan];
(1R,2S,2'R,4R,5'S)-2'-Ethyl-1,5',7,7-tetramethyl-spiro[bicyclo[2.2.1]heptan-2,4'-[1,3]dioxan];
(1R,2S,2'S,4R,5'S)-2'-Isopropyl-1,5',7,7-tetramethylspiro[bicyclo[2.2.1]heptan-2,4'-[1,3]-dioxan];
(1R,2S,2'R,4R,5'S)-2'-Isopropyl-1,5',7,7-tetramethylspiro[bicyclo[2.2.1]heptan-2,4'-[1,3]-dioxan];
(1R,2S,2'S,4R,5'R)-2'-Isopropyl-1,5',7,7-tetramethylspiro[bicyclo[2.2.1]heptan-2,4'-[1,3]-dioxan];
(1R,2S,4R,5'S)-1,2',2',5',7,7-Hexamethylspiro-[bicyclo[2.2.1]heptan-2,4'-[1,3]dioxan];
(1R,2S,4R,5'R)-1,2',2',5',7,7-Hexamethylspiro-[bicyclo[2.2.1]heptan-2,4'-[1,3]dioxan];
(1RS,2SR,2'RS/SR,4RS,5'RS/SR,6'RS/SR)-1,2',5',6',7,7-Hexamethylspiro[bicyclo[2.2.1]-heptan-2,4'-[1,3]dioxan];
(1RS,2SR,4RS,5'RS/SR,6'RS/SR)-1,2',2',5',6',7,7-Heptamethylspiro[bicyclo[2.2.1]heptan-2,4'-[1,3]-dioxan];
(1RS,2SR,2'RS/SR,4RS,5'RS/SR,6'RS/SR)-6'-Ethyl-1,2',5',7,7-pentamethylspiro[bicyclo[2.2.1]heptan-2,4'-[1,3]dioxan];
(1RS,2SR,4RS,5'RS/SR,6'RS/SR)-6'-Ethyl-1,2',2',5',7,7-hexamethylspiro[bicyclo[2.2.1]-heptan-2,4'-[1,3]dioxan] und
(1RS,2SR,2'RS/SR,4RS,5'RS/SR)-5'-Ethyl-1,2',7,7-tetramethylspiro[bicyclo[2.2.1]heptan-2,4'-[1,3]dioxan].

4. Duftstoffzusammensetzung, umfassend eine Verbindung nach einem der vorhergehenden Ansprüche.

5. Verwendung einer Verbindung nach einem der Ansprüche 1 bis 3 als Duftstoffbestandteil.

6. Verfahren zur Herstellung einer Duftstoffanwendung, bei dem man eine wirksame Menge einer Verbindung der Formel (1) nach einem der Ansprüche 1 bis 3 einarbeitet.

## Revendications

1. Composé de formule (1) 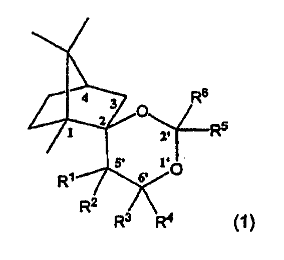 dans laquelle R¹ à R⁶ représentent indépendamment un atome d'hydrogène ; un groupe alkyle en C₁₋₄ ; ou un groupe alcényle en C₂₋₄ ; ou un groupe cycloalkyle en C₃₋₄ ; ou au moins l'un des résidus R¹ et R², R³ et R⁴, et/ou R⁵ et R⁶ forment, conjointement avec l'atome de carbone auquel ils sont fixés, un noyau cycloalkyle en C₃₋₅ ;
à condition que R¹ soit un groupe alkyle en C₁₋₄, un groupe alcényle en C₂₋₄ ou un groupe cycloalkyle en C₃₋₄ si R² est un atome d'hydrogène ; et que le nombre d'atomes de carbone de R¹+R²+R³+R⁴+R⁵+R⁶ soit compris entre 1 et 6.

2. Composé selon la revendication 1, dans lequel R² et R⁶ sont un atome d'hydrogène et le composé de formule (1) est enrichi en un composé de formule (1') 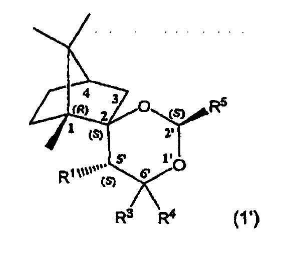 dans laquelle R¹ et R⁵ sont un groupe alkyle en C₁₋₄ ; ou un groupe alcényle en C₂₋₄ ; ou un groupe cycloalkyle en C₃₋₄ ; et
R³ et R⁴ présentent la même signification que celle indiquée dans la revendication 1.

3. Composé selon la revendication 1, choisi parmi le groupe constitué
du (1R,2S,2'S,4R,5'S)-1,2',5',7,7-pentaméthylspiro[bicyclo[2.2.1]heptane-2,4'-[1,3]dioxane],
du (1R,2S,2'R,4R,5'S)-1,2',5',7,7-pentaméthylspiro[bicyclo[2.2.1]heptane-2,4'-[1,3]dioxane],
du (1R,2S,2'S,4R,5'R)-1,2',5',7,7-pentaméthylspiro[bicyclo[2.2.1]heptane-2,4'-[1,3]dioxane],
du (1R,2S,2'S,4R,5'S)-2'-éthyl-1,5',7,7-tétraméthylspiro[bicyclo[2.2.1]heptane-2,4'-[1,3]dioxane],
du (1R,2S,2'R,4R,5'S)-2'-éthyl-1,5',7,7-tétraméthylspiro[bicyclo[2.2.1]heptane-2,4'-[1,3]dioxane],
du (1R,2S,2'S,4R,5'S)-2'-isopropyl-1,5',7,7-tétraméthylspiro[bicyclo[2.2.1]heptane-2,4'-[1,3]dioxane],
du (1R,2S,2'R,4R,5'S)-2'-isopropyl-1,5',7,7-tétraméthylspiro[bicyclo[2.2.1]heptane-2,4'-[1,3]dioxane],
du (1R,2S,2'S,4R,5'R)-2'-isopropyl-1,5',7,7-tétraméthylspiro[bicyclo[2.2.1]heptane-2,4'-[1,3]dioxane],
du (1R,2S,4R,5'S)-1,2',2',5',7,7-hexaméthylspiro[bicyclo[2.2.1]heptane-2,4'-[1,3]dioxane],
du (1R,2S,4R,5'R)-1,2',2',5',7,7-hexaméthylspiro[bicyclo[2.2.1]heptane-2,4'-[1,3]dioxane],
du (1RS,2SR,2'RS/SR,4RS,5'RS/SR,6'RS/SR)-1,2',5',6',7,7-hexaméthylspiro[bicyclo[2.2.1]heptane-2,4'-[1,3]dioxane],
du (1RS,2SR,4RS,5'RS/SR,6'RS/SR)-1,2',2',5',6',7,7-heptaméthylspiro[bicyclo[2.2.1]heptane-2,4'-[1,3]dioxane],
du (1RS,2SR,2'RS/SR,4RS,5'RS/SR,6'RS/SR)-6'-[ éthyl-1,2',5',7,7-pentaméthylspiro[bicyclo[2.2.1]heptane-2,4'-[1,3]dioxane],
du (1RS,2SR,4RS,5'RS/SR,6'RS/SR)-6'-éthyl-1,2',2',5',7,7-hexaméthylspiro[bicyclo[2.2.1]heptane-2,4'-[1,3]dioxane] et
du (1RS,2SR,2'RS/SR,4RS,5'RS/SR)-5'-éthyl-1,2',7,7-tétraméthylspiro[bicyclo[2.2.1]heptane-2,4'-[1,3]dioxane].

4. Composition de fragrance comprenant un composé tel que défini dans l'une quelconque des revendications précédentes.

5. Utilisation, en tant qu'ingrédient de fragrance, d'un composé tel que défini dans l'une quelconque des revendications 1 à 3.

6. Procédé de fabrication d'une application de fragrance, comprenant l'incorporation d'une quantité efficace d'un composé de formule (1) tel que défini dans l'une quelconque des revendications 1 à 3.
